(19) **Europäisches Patentamt**
**European Patent Office**
**Office européen des brevets**

(11) **EP 4 075 387 B1**

(12) **EUROPEAN PATENT SPECIFICATION**

(45) Date of publication and mention
of the grant of the patent:
**08.05.2024  Bulletin 2024/19**

(21) Application number: **20911035.2**

(22) Date of filing: **25.12.2020**

(51) International Patent Classification (IPC):
**G06T 17/00** *(2006.01)*      **A61F 2/28** *(2006.01)*
**A61F 2/30** *(2006.01)*

(52) Cooperative Patent Classification (CPC):
**G06T 17/00; A61F 2/28; A61F 2/3094;**
A61F 2/30942; A61F 2002/2835; A61F 2002/3092;
G06T 2210/41

(86) International application number:
**PCT/CN2020/139464**

(87) International publication number:
**WO 2021/136094 (08.07.2021 Gazette 2021/27)**

(54) **POROSITY INFORMATION PROCESSING METHOD AND DEVICE FOR BONE SCAFFOLD MODEL**

VERFAHREN UND VORRICHTUNG ZUR VERARBEITUNG VON POROSITÄTSINFORMATIONEN
FÜR KNOCHENGERÜSTMODELL

PROCÉDÉ ET DISPOSITIF DE TRAITEMENT D'INFORMATIONS DE POROSITÉ POUR MODÈLE
D'ÉCHAFAUDAGE OSSEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **03.01.2020  CN 202010005911**

(43) Date of publication of application:
**19.10.2022  Bulletin 2022/42**

(73) Proprietor: **Shanghai Jiao Tong University**
**Shanghai 200240 (CN)**

(72) Inventors:
  • **CHEN, Xiaojun**
    **Shanghai 200240 (CN)**
  • **SHI, Haochen**
    **Shanghai 200240 (CN)**
  • **WANG, Enpeng**
    **Shanghai 200240 (CN)**
  • **FANG, Jun**
    **Shanghai 200240 (CN)**

(74) Representative: **Sun, Yiming**
**HUASUN Patent- und Rechtsanwälte**
**Friedrichstraße 33**
**80801 München (DE)**

(56) References cited:
  CN-A- 102 087 676      CN-A- 102 973 334
  CN-A- 109 622 958      CN-A- 111 223 179
  CN-B- 102 024 076      ES-A1- 2 578 705
  JP-A- 2018 038 848      US-A1- 2014 363 481

• **PAPAZETIS G ET AL: "Direct porous structure
generation of tissue engineering scaffolds for
layer-based additive manufacturing", THE
INTERNATIONAL JOURNAL OF ADVANCED
MANUFACTURING TECHNOLOGY, SPRINGER,
LONDON, vol. 86, no. 1, 29 December 2015
(2015-12-29), pages 871-883, XP036028051, ISSN:
0268-3768, DOI: 10.1007/S00170-015-8237-1
[retrieved on 2015-12-29]**
• **Wei Sun: "Computer Aided Tissue Engineering:
Application to Biomimetic Modeling and Design
of Tissue Scaffold", Journal of Medical
Biomechanics, vol. 20, no. 4, 30 December 2005
(2005-12-30), pages 247-255, XP055826715,**

**Description**

**FIELD OF TECHNOLOGY**

The present invention relates to a computer technology, in particular to a perforation information processing method and device for a bone scaffold model.

Specifically, the present invention relates to a perforation information processing method and device for a bone scaffold model as defined in claim 1 and claim 4 attached, respectively.

**BACKGROUND**

The article "Direct porous structure generation of tissue engineering scaffolds for layer-based additive manufacturing" by G. Papazetis et al., Int. J. Adv. Manuf. Technol. (2016) 86:871, discloses a perforation information processing method and device for a bone scaffold model of the generic type as defined above. Specifically, this article discloses a modeling algorithm which exploits a macro-pore as a periodically repeated unit cell, designing different types of unit cells on a CAD system to populate a library. All unit cells lying on an outer boundary of a scaffold are represented in their exact shapes. The rest of the unit cells, lying totally inside the scaffold boundary and constituting a vast majority of the total umber of unit cells, are represented in essence by a reference point and a reference direction.

Document ES 2 578 705 A discloses a macroporous scaffolding for bone tissue engineering. The method disclosed is based on the Voronoi method, to define an interconnected and variable macroporosity scaffold from the trabecular thickness of non-constant section, the trabecular separation, the number of Voronoi nucleation points and the volume of the area to be filled of the bone defect, the scaffold to be manufactured by three-dimensional printing with biocompatible materials.

Document JP 2018-038848 A discloses improved randomized porous structures and methods of manufacturing such porous structures, wherein a scaffold of such porous structure is formed by dividing the space between a plurality of spatial coordinates of a defined volume, where the plurality of spatial coordinates are moved in a random direction by a random finite distance according to a predetermined randomization limit.

Document CN 102 024 076 A discloses a modeling method for a micropore structure in a bionic bone scaffold, comprising the steps of: a negative model of the micropore structure in the bionic bone scaffold is solved on the basis of a multi-constraint knapsack problem model by using an ellipsoid as a base element, using the minimum accommodating box of a bone scaffold model as a constraint space and using a hybrid genetic algorithm; and then a bionic bone scaffold model comprising the micropore structure is built through a Boolean subtraction operation between the scaffold model which does not contain micropores and the negative model of the micropore structure.

In bone defect repair treatment, to repair the damaged bone structure, a bone scaffold needs to be designed and surgically implanted into the patient's body. With the progress of research, it has been found that a bone scaffold with a porous structure can be combined with the host bone in a biological form, and the porosity, the pore size and the pore pattern of the porous structure of the bone scaffold all affect the combination in the biological form.

In the related art, methods of material science are mostly used to manufacture bone scaffolds with a porous structure through a special process, but the internal structures of the porous bone scaffolds generated by this method are uncertain.

Many researchers use computer-aided modeling methods to design bone scaffolds with a porous structure, and some of them use the three-dimensional model Boolean operation method to cut infarctate bone scaffold models by using a long cylinder or long rectangle from all directions. However, these methods require a large quantity of computation and are difficult to parallelize.

**SUMMARY**

An objective of the present invention is to overcome the defects in the related art, thereby providing a perforation information processing method and device for a bone scaffold model.

In view of this object, there are provided, in accordance with the present invention, a perforation information processing method for a bone scaffold model according to claim 1 attached and a perforation information processing device for a bone scaffold model according to claim 4 attached. Preferred embodiments of the invention are provided by the dependent claims attached.

Compared with the related art, the present invention has at least one of the following advantages.

1) Users have great autonomy in design and can completely customize the lattice structure.

2) The core computation steps can be highly parallelizable, which can greatly reduce the time required for computation.

**BRIEF DESCRIPTION OF THE DRAWINGS**

FIG. 1 is a schematic flowchart of main steps of a method according to the present invention;

FIG. 2 is a schematic diagram of a nonporous bone scaffold model;

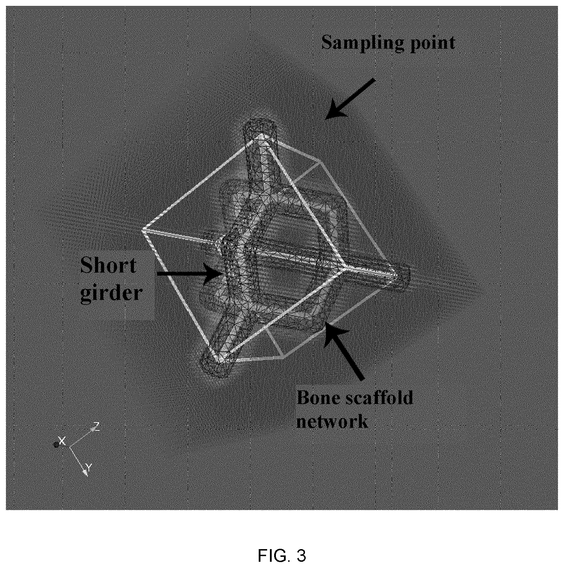

FIG. 3 is a structural diagram of a lattice in a porous bone scaffold; and

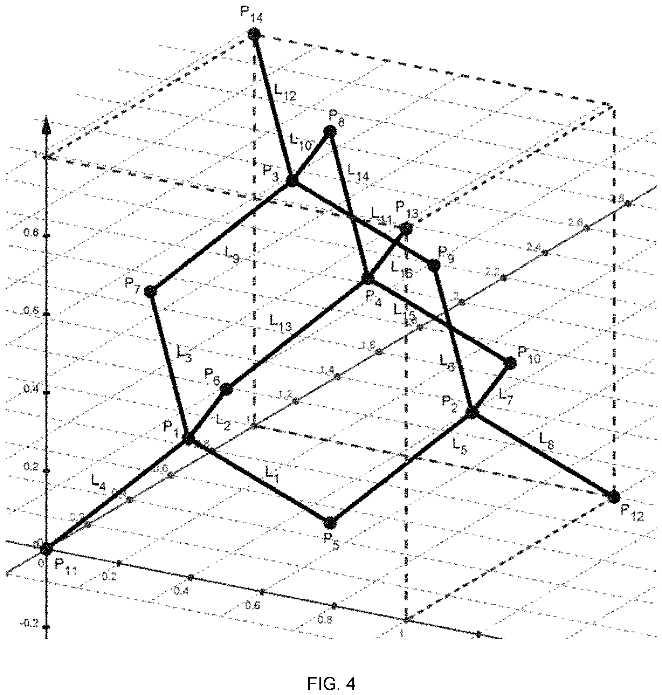

FIG. 4 is a specific structural diagram of a custom lattice.

**DETAILED DESCRIPTION OF THE EMBODIMENTS**

The present invention is described in detail below with reference to the accompanying drawings and specific embodiments. This embodiment is implemented on the premise of the technical solution of the present invention, and provides detailed implementations and a specific operation process, but the protection scope of the present invention is not limited to the following embodiments.

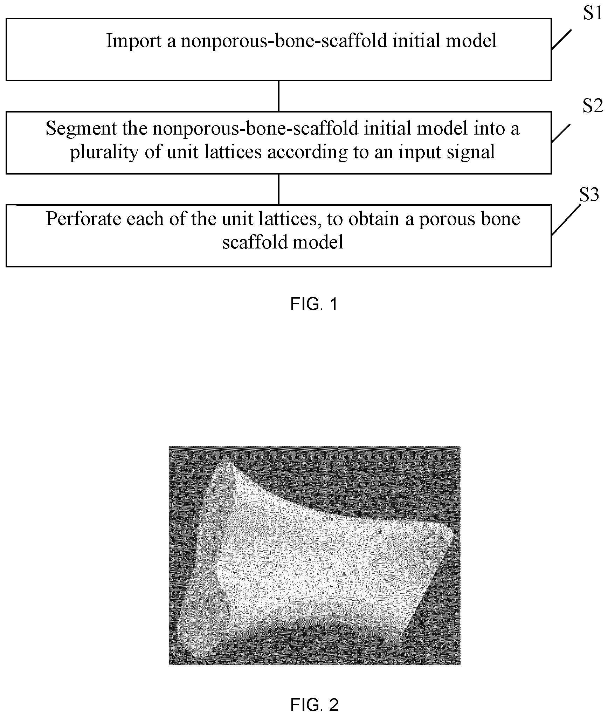

As shown in FIG. 1, a perforation information processing method for a bone scaffold model is implemented in a manner of computer software, including:

step S1: Import a nonporous-bone-scaffold initial model, shown in FIG. 2, with a following coordinate range: $X$:[-46.9, -31.5], $Y$:[18.9,44.1],$Z$: [42.8,70.1];

step S2: segmenting the nonporous-bone-scaffold initial model into a plurality of unit lattices according to an input signal; and

step S3: perforating each of the unit lattices, to obtain a porous bone scaffold model.

For a single unit lattice, step S3 specifically includes the following steps:
step S31: Draw a first quantity of short girders in the unit lattice, to form a porous lattice template, where the first quantity is received from an input device. Step S31 specifically includes:

step S311: Select a plurality of key points in a cube range from coordinates (0, 0, 0) to (1, 1, 1), where 14 key points are specified as follows in this embodiment;

$$P_1\left(\frac{1}{4},\frac{1}{4},\frac{1}{4}\right), P_2\left(\frac{3}{4},\frac{3}{4},\frac{1}{4}\right), P_3\left(\frac{1}{4},\frac{3}{4},\frac{3}{4}\right), P_4\left(\frac{3}{4},\frac{1}{4},\frac{3}{4}\right), P_5\left(\frac{1}{2},\frac{1}{2},0\right)$$

$$P_6\left(\frac{1}{2},0,\frac{1}{2}\right), P_7\left(0,\frac{1}{2},\frac{1}{2}\right), P_8\left(\frac{1}{2},\frac{1}{2},1\right), P_9\left(\frac{1}{2},1,\frac{1}{2}\right), P_{10}\left(1,\frac{1}{2},\frac{1}{2}\right)$$

$$P_{11}(0,0,0), P_{12}(1,1,0), P_{13}(1,0,1), P_{14}(0,1,1)$$

step S312: Create line segments between the generated key points, to form the short girders in the unit lattice, where 16 line segments are created as follows in this embodiment; and

$$L_1 = \overline{P_1P_5}, L_2 = \overline{P_1P_6}, L_3 = \overline{P_1P_7}, L_4 = \overline{P_1P_{11}}, L_5 = \overline{P_2P_5}, L_6 = \overline{P_2P_9}$$

$$L_7 = \overline{P_2P_{10}}, L_8 = \overline{P_2P_{12}}, L_9 = \overline{P_3P_7}, L_{10} = \overline{P_3P_8}, L_{11} = \overline{P_3P_9}, L_{12} = \overline{P_3P_{14}}$$

$$L_{13} = \overline{P_4P_6}, L_{14} = \overline{P_4P_8}, L_{15} = \overline{P_4P_{10}}, L_{16} = \overline{P_4P_{13}}$$

step S313: Check whether there is a key point, of which a connectivity is still 1 after the lattices are laid densely, among the key points generated in step S21 in the structural diagram of the line segments generated in step S22, and return to step S21 to regenerate key points if there is, otherwise, perform step S32.

Step S32: Generate equidistant sampling points according to a specified sampling pitch in a model space in which the nonporous-bone-scaffold initial model is located;

In this embodiment, the sampling pitch $\Delta L = 0.1$ is determined according to the model dimension $\Delta X = 15.4$, $\Delta Y = 25.2$, $\Delta Z = 27.3$ of the nonporous-bone-scaffold initial model, a specified lattice dimension $L_{Lattice} = 2.5$, and a specified short girder radius $R_{Girder} = 0.3$.

Then, according to the determined sampling pitch $\Delta L = 0.1$, a total of $N = 13867104$ sampling points are created in a sampling range $X$: [-47.9,-30.5], $Y$:[17.9,45.1],$Z$: [41.8,71.1] of the model space where the nonporous-bone-scaffold initial model is located.

Step S33: Calculate shortest distances between each of the sampling points and all the short girders. Step S33 specifically includes:

step S331: Scale a dimension of the unit lattice to a specified dimension, where in this embodiment, the dimension is enlarged from $1 \times 1 \times 1$ to $2.5 \times 2.5 \times 2.5$, and densely fill the scaled unit lattices in the model space;

step S332: Assign an initial value $+\infty$ to distances $D_{X_iY_iZ_i}$ of the $N$ sampling points generated in step S32 in a distance field;

step S333: traversing all lattices that are filled in step S331 and that possibly contribute to the shortest distances of the sampling points generated in step S3 in the distance field; and

step S334: Traverse short girders in all the lattices traversed in step S333, use an OBBTree data structure to speed up a determination of a positional relationship between the short girder and the non-porous model designed in step S1, if the short girder is completely inside the nonporous-bone-scaffold initial model, update shortest distance values of sampling points in a specified range near the short girder, if a part of the short girder is inside the nonporous-bone-scaffold initial model, calculate the part of the short girder inside the nonporous-bone-scaffold initial model, and update the shortest distance values $D_{X_iY_iZ_i}$ of the sampling points in a specific range nearby the part for the part.

Values in each region in step S334 are updated concurrently and independently.

Step S34: Use a Marching-Cubes algorithm to extract an isosurface of which a value is $R_{Girder} = 0.3$ in the shortest distance values $D_{X_iY_iZ_i}$, that is, to obtain a final porous-bone-scaffold initial model.

**Claims**

1. A perforation information processing method for a bone scaffold model, comprising:

   step S1: importing a nonporous-bone-scaffold initial model;
   step S2: segmenting the nonporous-bone-scaffold initial model into a plurality of unit lattices according to an input signal; and
   step S3: perforating each of the unit lattices, to obtain a porous bone scaffold model, **characterized in that** for a single unit lattice, step S3 specifically comprises:

      step S31: drawing a first quantity of short girders in the unit lattice to form a porous lattice template, wherein the first quantity is received from an input device;
      step S32: generating equidistant sampling points according to a specified sampling pitch in a model space in which the nonporous-bone-scaffold initial model is located;
      step S33: calculating shortest distances between each of the sampling points and all the short girders; and
      step S34: extracting, according to the generated sampling points, an isosurface according to a specified short girder cylinder radius by using an isosurface extraction algorithm, to perforate the unit lattices.

2. The perforation information processing method for a bone scaffold model according to claim 1, wherein step S33 specifically comprises:

> step S331: scaling a dimension of the unit lattice to a specified dimension, and densely filling the scaled unit lattices in the model space;
> step S332: assigning an initial value $+\infty$ to distances of all the sampling points generated in step S32 in a distance field;
> step S333: traversing all lattices that are filled in step S331 and that possibly contribute to the shortest distances of the sampling points generated in step S3 in the distance field; and
> step S334: traversing short girders in all the lattices traversed in step S333, if the short girder is completely inside the nonporous-bone-scaffold initial model, updating shortest distance values of sampling points in a specified range near the short girder, if a part of the short girder is inside the nonporous-bone-scaffold initial model, calculating the part of the short girder inside the nonporous-bone-scaffold initial model, and updating the shortest distance values of the sampling points in a specific range nearby the part for the part.

3. The perforation information processing method for a bone scaffold model according to claim 2, wherein values in each region in step S334 are updated concurrently and independently.

4. A perforation information processing device for a bone scaffold model, comprising a processor, a memory, and a program stored in the memory and performed by the processor, wherein when performing the program, the processor performs the following steps:

> step S1: importing a nonporous-bone-scaffold initial model;
> step S2: segmenting the nonporous-bone-scaffold initial model into a plurality of unit lattices according to an input signal; and
> step S3: perforating each of the unit lattices, to obtain a porous bone scaffold model, **characterized in that** for a single unit lattice, step S3 specifically comprises:
>
>> step S31: drawing a first quantity of short girders in the unit lattice, to form a porous lattice template, wherein the first quantity is received from an input device;
>> step S32: generating equidistant sampling points according to a specified sampling pitch in a model space in which the nonporous-bone-scaffold initial model is located;
>> step S33: calculating shortest distances between each of the sampling points and all the short girders; and
>> step S34: extracting, according to the generated sampling points, an isosurface according to a specified short girder cylinder radius by using an isosurface extraction algorithm, to perforate the unit lattices.

5. The perforation information processing device for a bone scaffold model according to claim 4, wherein step S33 specifically comprises:

> step S331: scaling a dimension of the unit lattice to a specified dimension, and densely filling the scaled unit lattices in the model space;
> step S332: assigning an initial value $+\infty$ to distances of all the sampling points generated in step S32 in a distance field;
> step S333: traversing all lattices that are filled in step S331 and that possibly contribute to the shortest distances of the sampling points generated in step S3 in the distance field; and
> step S334: traversing short girders in all the lattices traversed in step S333, if the short girder is completely inside the nonporous-bone-scaffold initial model, updating shortest distance values of sampling points in a specified range near the short girder, if a part of the short girder is inside the nonporous-bone-scaffold initial model, calculating the part of the short girder inside the nonporous-bone-scaffold initial model, and updating the shortest distance values of the sampling points in a specific range nearby the part for the part.

6. The perforation information processing device for a bone scaffold model according to claim 5, wherein values in each region in step S334 are updated concurrently and independently.

**Patentansprüche**

1. Verfahren zur Verarbeitung von Perforationsinformationen für ein Knochengerüstmodell, das folgende Schritte umfasst:

Schritt S1: Importieren eines nichtporösen, anfänglichen Knochengerüstmodells;

Schritt S2: Segmentieren des nichtporösen, anfänglichen Knochengerüstmodells in mehrere Einheitsgitter entsprechend einem Eingangssignal; und

Schritt S3: Perforieren jedes der Einheitsgitter, um ein poröses Knochengerüstmodell zu erhalten, **dadurch gekennzeichnet, dass**

für ein einzelnes Einheitsgitter der Schritt S3 spezifisch umfasst:

Schritt S31: Ziehen einer ersten Menge von kurzen Trägern in das Einheitsgitter, um eine poröse Gittervorlagenschablone zu bilden, wobei die erste Menge von einer Eingangsvorrichtung empfangen wird;

Schritt S32: Erzeugen gleichmäßig beabstandeter Probepunkte entsprechend einem spezifizierten Probenzwischenraum in einem Modellraum, in dem das nichtporöse, anfängliche Knochengerüstmodell angeordnet ist;

Schritt S33: Berechnen der kürzesten Abstände zwischen jedem der Probepunkte und all den kurzen Trägern; und

Schritt S34: entsprechend der erzeugen Probepunkte Extrahieren einer Isooberfläche entsprechend einem spezifizierten kurzen Trägerzylinderradius, indem ein Isooberflächenextraktionsalgorithmus verwendet wird, um die Einheitsgitter zu perforieren.

2. Verfahren zur Verarbeitung von Perforationsinformationen für ein Knochengerüstmodell nach Anspruch 1, wobei der Schritt S33 spezifisch umfasst:

Schritt S331: Skalierung einer Dimension des Einheitsgitters auf eine spezifizierte Dimension, und ein dichtes Auffüllen der skalierten Einheitsgitter in dem Modellraum;

Schritt S332: Zuweisung eines Anfangswerts $+\infty$ zu den Abständen von allen Probepunkten, die im Schritt S32 in einem Abstandsfeld erzeugt worden sind;

Schritt S333: Überqueren aller Gitter, die im Schritt S331 aufgefüllt worden sind, und die möglicherweise zu den kürzesten Abständen der Probepunkte, die im Schritt S3 in dem Abstandsfeld erzeugt worden sind, beitragen; und

Schritt S334: ein Überqueren der kurzen Träger in all den Gittern, die im Schritt S333 überquert worden sind, wenn der kurze Träger vollständig innerhalb des nichtporösen, anfänglichen Knochengerüstmodells liegt, ein Aktualisieren der kürzesten Abstandswerte der Probepunkte in einem spezifizierten Bereich in der Nähe des kurzen Trägers, wenn ein Teil des kurzen Trägers innerhalb des nichtporösen, anfänglichen Knochengerüstmodells liegt, ein Berechnen des Teils des kurzen Trägers innerhalb des nichtporösen, anfänglichen Knochengerüstmodells, und ein Aktualisieren der kürzesten Abstandswerte der Probepunkte in einem spezifizierten Bereich in der Nähe des Teils für das Teil.

3. Verfahren zur Verarbeitung von Perforationsinformationen für ein Knochengerüstmodell nach Anspruch 2, wobei die Werte in jedem Bereich im Schritt S334 laufend und unabhängig aktualisiert werden.

4. Vorrichtung zur Verarbeitung von Perforationsinformationen für ein Knochengerüstmodell, die einen Prozessor, einen Speicher und ein Programm umfasst, das in dem Speicher gespeichert ist und mittels des Prozessors ausgeführt wird, wobei dann, wenn das Programm ausgeführt wird, der Prozessor die folgenden Schritte ausführt:

Schritt S1: Importieren eines nichtporösen, anfänglichen Knochengerüstmodells;

Schritt S2: Segmentieren des nichtporösen, anfänglichen Knochengerüstmodells in mehrere Einheitsgitter entsprechend einem Eingangssignal; und

Schritt S3: Perforieren jedes der Einheitsgitter, um ein poröses Knochengerüstmodell zu erhalten, **dadurch gekennzeichnet, dass**

für ein einzelnes Einheitsgitter der Schritt S3 spezifisch umfasst:

Schritt S31: Ziehen einer ersten Menge von kurzen Trägern in das Einheitsgitter, um eine poröse Gittervorlagenschablone zu bilden, wobei die erste Menge von einer Eingangsvorrichtung empfangen wird;

Schritt S32: Erzeugen gleichmäßig beabstandeter Probepunkte entsprechend einem spezifizierten Probenzwischenraum in einem Modellraum, in dem das nichtporöse, anfängliche Knochengerüstmodell angeordnet ist;

Schritt S33: Berechnen der kürzesten Abstände zwischen jedem der Probepunkte und all den kurzen Trägern; und

Schritt S34: entsprechend der erzeugen Probepunkte Extrahieren einer Isooberfläche entsprechend einem spezifizierten kurzen Trägerzylinderradius, indem ein Isooberflächenextraktionsalgorithmus verwendet wird, um die Einheitsgitter zu perforieren.

5. Vorrichtung zur Verarbeitung von Perforationsinformationen für ein Knochengerüstmodell nach Anspruch 4, wobei der Schritt S33 spezifisch umfasst:

Schritt S331: Skalierung einer Dimension des Einheitsgitters auf eine spezifizierte Dimension, und ein dichtes Auffüllen der skalierten Einheitsgitter in dem Modellraum;
Schritt S332: Zuweisung eines Anfangswerts $+\infty$ zu den Abständen von allen Probepunkten, die im Schritt S32 in einem Abstandsfeld erzeugt worden sind;
Schritt S333: Überqueren aller Gitter, die im Schritt S331 aufgefüllt worden sind, und die möglicherweise zu den kürzesten Abständen der Probepunkte, die im Schritt S3 in dem Abstandsfeld erzeugt worden sind, beitragen; und
Schritt S334: ein Überqueren der kurzen Träger in all den Gittern, die im Schritt S333 überquert worden sind, wenn der kurze Träger vollständig innerhalb des nichtporösen, anfänglichen Knochengerüstmodells liegt, ein Aktualisieren der kürzesten Abstandswerte der Probepunkte in einem spezifizierten Bereich in der Nähe des kurzen Trägers, wenn ein Teil des kurzen Trägers innerhalb des nichtporösen, anfänglichen Knochengerüstmodells liegt, ein Berechnen des Teils des kurzen Trägers innerhalb des nichtporösen, anfänglichen Knochengerüstmodells, und ein Aktualisieren der kürzesten Abstandswerte der Probepunkte in einem spezifizierten Bereich in der Nähe des Teils für das Teil.

6. Vorrichtung zur Verarbeitung von Perforationsinformationen für ein Knochengerüstmodell nach Anspruch 5, wobei die Werte in jedem Bereich im Schritt S334 laufend und unabhängig aktualisiert werden.

**Revendications**

1. Procédé de traitement d'information de perforation pour un modèle d'échafaudage osseux, comprenant :

étape S1 : importer un modèle initial d'échafaudage osseux non-poreux ;
étape S2 : segmenter le modèle initial d'échafaudage osseux non-poreux en une pluralité de treillages unitaires selon un signal d'entrée ; et
étape S3 : perforer chacun des treillages unitaires, pour obtenir un modèle d'échafaudage osseux poreux, **caractérisé en ce que**
pour un treillage unitaire unique, l'étape S3 comprend spécifiquement :

étape S31 : dessiner une première quantité de poutrelles courtes dans le treillage unitaire pour former un gabarit de treillage poreux, dans lequel la première quantité est reçue d'un dispositif d'entrée ;
étape S32 : générer des points d'échantillonnage équidistants selon un pas d'échantillonnage spécifié dans un espace de modèle dans lequel le modèle initial d'échafaudage osseux non-poreux est situé ;
étape S33: calculer des distances les plus courtes entre chacun des points d'échantillonnage et toutes les poutrelles courtes ; et
étape S34 : extraire, selon les points d'échantillonnage générés, une iso-surface selon un rayon de cylindre de poutrelle courte spécifié en utilisant un algorithme d'extraction d'iso-surface, pour perforer les treillages unitaires.

2. Procédé de traitement d'information de perforation pour un modèle d'échafaudage osseux selon la revendication 1, dans lequel l'étape S33 comprend spécifiquement :

étape S331 : mettre à l'échelle une dimension du treillage unitaire à une dimension spécifiée et remplir de manière dense les treillages unitaires mis à l'échelle dans l'espace de modèle ;
étape S332 : assigner une valeur initiale $+\infty$ à des distances de tous les points d'échantillonnage générés à l'étape S32 dans un champ de distance ;
étape S333 : traverser tous les treillages qui sont remplis à l'étape S331 et qui contribuent peut-être aux distances les plus courtes des points d'échantillonnage générés à l'étape S3 dans le champ de distance ; et
étape S334 : traverser des poutrelles courtes dans tous les treillages traversés à l'étape S333, si le treillage court est complètement à l'intérieur du modèle initial d'échafaudage osseux non-poreux, mettre à jour des valeurs de distances les plus courtes de points d'échantillonnage dans une plage spécifiée près de la poutrelle courte, si une partie de la poutrelle courte est à l'intérieur du modèle initial d'échafaudage osseux non-poreux, calculer la partie de la poutrelle courte à l'intérieur du modèle initial d'échafaudage osseux non-poreux et mettre à jour les valeurs de distances les plus courtes des points d'échantillonnage dans une plage spécifique près de la partie pour la partie.

3. Procédé de traitement d'information de perforation pour un modèle d'échafaudage osseux selon la revendication 2, dans lequel des valeurs dans chaque région à l'étape S334 sont mises à jour simultanément et indépendamment.

4. Dispositif de traitement d'information de perforation pour un modèle d'échafaudage osseux, comprenant un processeur, une mémoire et un programme stocké dans la mémoire et exécuté par le processeur, dans lequel lors de l'exécution du programme, le processeur exécute les étapes suivantes :

   étape S1 : importer un modèle initial d'échafaudage osseux non-poreux ;
   étape S2 : segmenter le modèle initial d'échafaudage osseux non-poreux en une pluralité de treillages unitaires selon un signal d'entrée ; et
   étape S3 : perforer chacun des treillages unitaires, pour obtenir un modèle d'échafaudage osseux poreux, **caractérisé en ce que**
   pour un treillage unitaire unique, l'étape S3 comprend spécifiquement :

       étape S31 : dessiner une première quantité de poutrelles courtes dans le treillage unitaire pour former un gabarit de treillage poreux, dans lequel la première quantité est reçue d'un dispositif d'entrée ;
       étape S32 : générer des points d'échantillonnage équidistants selon un pas d'échantillonnage spécifié dans un espace de modèle dans lequel le modèle initial d'échafaudage osseux non-poreux est situé ;
       étape S33: calculer des distances les plus courtes entre chacun des points d'échantillonnage et toutes les poutrelles courtes ; et
       étape S34 : extraire, selon les points d'échantillonnage générés, une iso-surface selon un rayon de cylindre de poutrelle courte spécifié en utilisant un algorithme d'extraction d'iso-surface, pour perforer les treillages unitaires.

5. Dispositif de traitement d'information de perforation pour un modèle d'échafaudage osseux selon la revendication 4, dans lequel l'étape S33 comprend spécifiquement :

   étape S331 : mettre à l'échelle une dimension du treillage unitaire à une dimension spécifiée et remplir de manière dense les treillages unitaires mis à l'échelle dans l'espace de modèle ;
   étape S332 : assigner une valeur initiale+- à des distances de tous les points d'échantillonnage générés à l'étape S32 dans un champ de distance ;
   étape S333 : traverser tous les treillages qui sont remplis à l'étape S331 et qui contribuent peut-être aux distances les plus courtes des points d'échantillonnage générés à l'étape S3 dans le champ de distance ; et
   étape S334 : traverser des poutrelles courtes dans tous les treillages traversés à l'étape S333, si le treillage court est complètement à l'intérieur du modèle initial d'échafaudage osseux non-poreux, mettre à jour des valeurs de distances les plus courtes de points d'échantillonnage dans une plage spécifiée près de la poutrelle courte, si une partie de la poutrelle courte est à l'intérieur du modèle initial d'échafaudage osseux non-poreux, calculer la partie de la poutrelle courte à l'intérieur du modèle initial d'échafaudage osseux non-poreux et mettre à jour les valeurs de distances les plus courtes des points d'échantillonnage dans une plage spécifique près de la partie pour la partie.

6. Dispositif de traitement d'information de perforation pour un modèle d'échafaudage osseux selon la revendication 5, dans lequel des valeurs dans chaque région à l'étape S334 sont mises à jour simultanément et indépendamment.

| Import a nonporous-bone-scaffold initial model | S1 |

| Segment the nonporous-bone-scaffold initial model into a plurality of unit lattices according to an input signal | S2 |

| Perforate each of the unit lattices, to obtain a porous bone scaffold model | S3 |

FIG. 1

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 2578705 A
- JP 2018038848 A

- CN 102024076 A

**Non-patent literature cited in the description**

- **G. PAPAZETIS et al.** Direct porous structure generation of tissue engineering scaffolds for layer-based additive manufacturing. *Int. J. Adv. Manuf. Technol,* 2016, vol. 86, 871